# EUROPÄISCHE PATENTSCHRIFT

(11) **EP 1 992 293 B1**
(45) Veröffentlichungstag und Bekanntmachung des Hinweises auf die Patenterteilung: **20.11.2013**
(21) Anmeldenummer: 08153533.8
(22) Anmeldetag: 28.03.2008
(51) Int. Cl.: A61B 10/06, A61B 17/28

(54) **Medizinisches Greifgerät**
Medical gripping device
Appareil médical de saisie

(30) Priorität: 14.03.2007 DE 102007000151
(43) Veröffentlichungstag der Anmeldung: 19.11.2008
(73) Patentinhaber: Ovesco Endoscopy AG, 72074 Tübingen (DE)
(72) Erfinder: Schurr, Marc. O., 72072 Tübingen (DE); Ho, Chi-Nghia, 70180 Stuttgart (DE); Kirschniak, Andreas, 41061 Moenchengladbach (DE); Anhöck, Gunnar, 72762 Reutlingen (DE)
(74) Vertreter: Winter, Brandl, Fürniss, Hübner, Röss, Kaiser, Polte - Partnerschaft

(56) Entgegenhaltungen:
- WO-A-01/66025
- WO-A-01/89391
- WO-A-99/30622
- DE-A1- 10 013 927
- DE-A1- 19 833 600
- DE-U1- 20 309 775
- US-A- 5 511 564
- US-A- 6 077 287
- US-A1- 2002 026 199
- US-B1- 6 440 085

## Beschreibung

### Technisches Gebiet

Die vorliegende Erfindung bezieht sich auf ein medizinisches Greifgerät mit mehreren Branchen, die einzeln steuerbar sind und insbesondere ein medizinisches Greifgerät gemäß dem Oberbegriff des Patentanspruchs 1.

### Stand der Technik

Heutzutage ist der Einsatz der flexiblen Endoskopie ein gängiger Standard für die diagnostische und therapeutische Behandlung von Erkrankungen des Magen-Darm-Trakts. Dabei wird ein flexibles Endoskop in natürliche Öffnungen des Körpers eingeführt, wie zum Beilspiel Mund und Anus. Da bei dieser Operationsmethode kein direkter Zugriff auf das zu operierende Gewebe o.a. möglich ist, müssen flexible endoskopische Instrumente verwendet werden. Hierbei kommen auch Greifinstrumente zum Einsatz, die beispielsweise Gewebeproben entnehmen können oder womit Gewebe gegriffen und manipuliert werden kann.

Im Stand der Technik gibt es ein Greifgerät, das einen hohlen flexiblen Schaft besitzt, an dessen vorderem Ende zwei Branchen drehbar befestigt sind. Die beiden Branchen bilden eine Art Maul, das sich zu dem vorderen Ende des Greifgeräts öffnen kann. An dem hinteren Ende jeder Branche ist über einen Hebelarm ein zug- und druckfestes Seil montiert. Die beiden Seile sind mit einem Ende eines Bowdenzugs verbunden, der durch den holen Schaft läuft und an seinem anderen Ende mit einem Griff verbunden ist. Mit der Betätigung des Griffs kann das Maul, das sich an dem anderen Ende des Greifgeräts befindet, geöffnet und geschlossen werden. Das ganze Greifgerät wird durch einen Arbeitskanal eines Endoskops in den Körper eingeführt.

Gemäß einem weiteren Instrument aus dem Stand der Technik besitzt ein Greifgerät einen flexiblen Schaft mit einem starren Steg (oder einem starren Maulteil) an einem Ende. An diesem starren Steg ist eine Branche angelenkt, die mittels eines Bowdenzugs bewegbar ist. Der Bowdenzug verläuft in dem flexiblen Schaft und ist an dem anderen Ende des Greifgeräts mit einem Griff verbunden. Mit der Betätigung des Griffs wird die Branche geöffnet und geschlossen. Dieses Greifgerät kann durch einen Arbeitskanal eines Endoskops eingeführt werden.

Die Greifgeräte aus dem Stand der Technik besitzen allerdings einige Nachteile. In der Praxis ist es oft erforderlich, Gewebeteile miteinander zu verbinden oder Öffnungen im Gewebe zu verschließen. Dabei ist es erforderlich, dass die entsprechenden Gewebeteile oder die gegenüberliegenden Randabschnitte der Öffnungen gegriffen und somit fixiert werden können. Ansonsten ist keine kontrollierte Verbindung oder kein kontrollierter Verschluss des Gewebes möglich. Wenn zum Beispiel eine Perforation nicht verschlossen werden kann, muss oftmals ein offener Zugriff vorgenommen werden, um Komplikationen zu vermeiden, die beispielsweise durch eintretende Keime verursacht werden. Da aber die Greifgeräte aus dem Stand der Technik nur jeweils ein Maul besitzen, ist es zwangsläufig erforderlich, dass mindestens zwei dieser Greifgeräte zum Einsatz kommen. Dies ist aber im Hinblick auf den dann erforderlichen (zweiten Zugang) zweiten Arbeitskanal im Endoskop oder ein zusätzliches zweites Endoskop problematisch durch die Enge der natürlichen Körperöffnungen, wie beispielsweise Speiseröhre, Dünn- und Dickdarm. In jüngster Zeit wird an neuen Operationsmethoden, den so genannten NOTES (Natural Orifice Transluminal Endoscopy Surgery) geforscht, bei der versucht wird, ohne einen Hautschnitt in Körperhöhlen wie den Bauchraum zu gelangen, um dort zu operieren. Dabei werden die Zugänge mit Hilfe des flexiblen Instruments durch beispielsweise Magen, Dickdarm oder Vagina geschaffen. Diese Zugänge gilt es nach der Operation effektiv zu verschließen. Das gelingt jedoch nur wenn die entsprechenden Randabschnitte gegriffen und somit fixiert werden können.

Die DE-OS 198 33 600 A1 offenbart eine medizinische Zange mit zwei unabhängig voneinander beweglichen Maulteilen, die zur Ausbildung von zwei Mäulern mit einem zentralen Steg zusammenwirken, der an einem distalen Ende eines Schafts fixiert ist. Die aus der DE-OS 198 33 600 A1 bekannte medizinische Zange entspricht hinsichtlich ihres konstruktiven Aufbaus im Wesentlichen dem vorstehend beschriebenen Stand der Technik, wobei der Schaft ein biegesteifer Stahlschaft ist, der zwar eine gewisse Biegeelastizität aufweist, jedoch eigentlich als starr zu bezeichnen ist. Auch sind zur Betätigungsübertragung Schubstangen 64, 66 innerhalb des Schafts vorgesehen, deren Steifigkeit ausreicht, Betätigungs-Schubkräfte auf die Maulteile zu übertragen.

### Darstellung der Erfindung

### Technische Aufgabe

Es ist daher eine Aufgabe der Erfindung, ein medizinisches Greifgerät vorzusehen, mit dem mindestens zwei Gewebeteile separat gegriffen und somit fixiert werden können, das eine höhere Funktionalität hat.

### Technische Lösung

Die Aufgabe der vorliegenden Erfindung wird mit einem medizinischen Greifgerät gemäß dem Patentanspruch 1 gelöst. Weiter vorteilhafte Weiterbildungen des medizinischen Greifgeräts sind Gegenstand der Unteransprüche.

Gemäß einem ersten Aspekt der vorliegenden Erfindung besitzt ein medizinisches Greifgerät einen flexiblen Schaft mit einem vorderen und einem hinteren Ende, einen Steg bestehend aus mindestens einem Stegelement, der an dem vorderen Ende des Schafts befestigt ist, mindestens zwei Branchen, die an dem Steg angelenkt sind, und mindestens zwei flexible Steuermechanismen, die in dem Schaft angeordnet sind. Bei diesem medizinischen Greifgerät ist jede einzelne Branche mittels eines eigenen Steuermechanismus gegenüber dem Steg bewegbar. Das heißt, das Greifgerät besitzt mindestens zwei Mäuler, die unabhängig voneinander geöffnet und geschlossen werden können. Somit können mindestens zwei Gewebeabschnitte separat gegriffen und fixiert werden. Der Schaft des Greifgeräts ist flexibel, sodass er möglichst geringe Rückstellkräfte erzeugt, wenn er aus seiner Ausgangsform heraus verformt wird. An dem vorderen Ende des Schafts kann auch ein kleiner Vorsprung vorgesehen sein, an dem der Steg und die Branchen befestigt sein können. Die Branchen können zum Beispiel Widerhaken oder Zähne aufweisen, sie können profiliert oder flach, gerade oder bogenförmig sein. Die Stegelemente können entsprechend so ausgebildet sein, dass sie im Zusammenspiel mit den Branchen ein herausrutschen des gegriffenen Gewebes aus dem jeweiligen Maul verhindern. Die Branchen und Stegelemente können auch insbesondere so ausgebildet sein, dass sie das gegriffene Gewebe nicht verletzen.

Gemäß einer vorteilhaften Ausführungsform besteht der Steg des medizinischen Greifgeräts aus mindestens zwei Stegelementen, wobei maximal eines der Stegelemente starr an dem Schaft befestigt ist und die restlichen Stegelemente drehbar an dem vorderen Ende des Schafts oder dem maximal einen starren Stegelement angelenkt sind. Das heißt, die Stegelemente sind entweder alle drehbar an dem vorderen Ende des Schafts angelenkt oder ein Stegelement ist starr mit dem vorderen Ende des Schafts verbunden und die restlichen Stegelemente sind drehbar an dem vorderen Ende des Schafts oder an dem einen starren Stegelement angelenkt. Zwischen oder an den Stegelementen ist mindestens ein Element so angeordnet, dass die beweglichen Stegelemente mit Hilfe des Elements zumindest in eine Richtung bewegt werden können. Darüber hinaus ist jede Branche des Greifgeräts an einem Stegelement drehbar angelenkt.

Gemäß einer vorteilhaften Ausführungsform des medizinischen Greifgeräts ist mindestens ein elastisches Element so an den Stegelementen angeordnet, dass es die Stegelemente an ihrem körperfernen Ende auseinander drängt. Es kann entweder ein elastisches Element vorgesehen sein, das alle Stegelemente auseinander drängt, oder es können mehrere elastische Elemente vorgesehen sein, die jeweils zwei Stegelemente auseinander drängen. Das mindestens eine elastische Element kann beispielsweise in dem jeweiligen Maul zwischen den Stegelementen angeordnet sein und die Stegelemente auseinander drücken. Dies ist beispielsweise durch Druckfedem oder Gummielemente möglich. Im Gegensatz dazu kann das mindestens eine elastische Element aber auch außerhalb der Mäuler angeordnet sein und die Stegelemente auseinander ziehen. Dies kann zum Beispiel durch eine bogenförmige Zugfeder realisiert werden. Anstelle eines elastischen Elements kann aber auch ein magnetisches Element diese Aufgabe übernehmen, wobei das magnetische Element in den Stegelementen enthalten sein kann. Des Weiteren könnte auch ein anderes Element wie beispielsweise eine Art mikroskopische Luftdruckfeder verwendet werden. Diese Elemente können alternativ auch zwischen den Stegelementen und dem Schaft des Greifgeräts angeordnet sein.

Alternativ zu dem vorstehend beschriebenen Aufbau, können aber in Zukunft beispielsweise elektrische Mikromotoren den Abstand zwischen den Stegelementen regulieren. Diese Ausführungsform kann aufgrund der schnellen Entwicklung auf diesem Gebiet bereits bald Anwendung finden.

Die Stegelemente können aber auch gänzlich anders ausgebildet sein. Zum Beispiel können die einzelnen beweglichen Stegelemente aus einem elastischen Material wie beispielsweise Federstahl ausgebildet sein. Diese Stegelemente werden dann an dem Ende, das dem Schaft zugewandt ist, so verbunden, dass sich die zu diesen verbundenen Enden entgegen gesetzten Enden der Stegelemente durch die ihnen eigene Elastizität voneinander entfernen.

Diese Ausbildung ist insbesondere dann sinnvoll, wenn das erfindungsgemäße medizinische Greifgerät im Zusammenspiel mit einem Endoskop verwendet wird. In diesem Fall wird das erfindungsgemäße Greifgerät durch einen Arbeitskanal des Endoskops eingeführt. Die Stegelemente, deren vordere Enden auf eine der zuvor beschriebenen Weisen das Bestreben haben, sich voneinander zu entfernen, können dies uneingeschränkt tun, so lange der gesamte Kopf des erfindungsgemäßen Greifgeräts, der aus den Branchen und den Stegelementen besteht, aus dem Arbeitskanal des Endoskops heraus ragt. Wenn aber der Kopf des Greifgeräts nun ein Stück weit in eine Hülse hinein gezogen wird, die sich an dem Ende des Endoskops befindet, liegen die Stegelemente an der Hülse an und werden bei einem weiteren Hineinziehen des Greifgeräts in die Hülse zu der Achse des Greifgeräts hin verformt. Es kann auch die Hülse bewegt bzw. aufgeschoben werden. Einzig wichtig hierbei ist die relative Bewegung von Greifgerät zu Hülse. Wenn der Kopf des Greifgeräts wieder vollständig aus der Hülse heraus geschoben wird, entfernen sich die vorderen Enden der Stegelemente wieder voneinander. Auf diese Weise kann der Abstand der vorderen Enden der Stegelemente und somit der inneren Ränder der Mäuler voneinander eingestellt werden. Somit kann der Abstand der Stegelemente dem Abstand der zu greifenden Gewebeteile angepasst werden. Die Hülse kann auch ein Teil eines Endoskops sein.

Erfindungsgemäß besitzen die Steuermechanismen jeweils ein zug- und druckfestes Übertragungselement. Die mindestens zwei Übertragungselemente verlaufen gemeinsam in dem Schaft, wobei jedes dieser Übertragungselemente an dem vorderen Ende des Schafts mit einer Branche verbunden ist. Zudem besitzen die Steuermechanismen jeweils eine Steuervorrichtung, die an dem hinteren Ende des Schafts vorgesehen ist und mit dem zugehörigen Übertragungselement verbunden ist. Jedes Übertragungselement überträgt die von der entsprechenden Steuervorrichtung ausgegebene Bewegung an die entsprechende Branche.

Des Weiteren sind die Übertragungselemente erfindungsgemäß Bowdenzüge und

die Steuervorrichtungen sind Griffelemente, wobei die Griffelemente betätigt werden, um die entsprechende Branche zu öffnen, und freigegeben werden, um die entsprechende Branche zu schließen. Die Griffelemente sind zum Beispiel so ausgebildet, dass jedes Griffelement aus einem U-förmigen Bügel aus Metall besteht und die Griffelemente einen Griff bilden oder an einem Griff vorgesehen sind. Ein Ende des Bügels ist beispielsweise an dem hinteren Ende des Schafts des Griffgeräts befestigt und an dem anderen Ende des Bügels ist ein Ende des entsprechenden Bowdenzugs montiert. Die Griffelemente bilden somit gemeinsam den Griff. Wenn das Ende des Bügels, an dem der Bowdenzug befestigt ist, zu dem anderen Ende des Bügels hin gedrückt wird, wird der Bowdenzug in den Schaft des Greifgeräts hinein gedrückt. Am anderen Ende des Schafts wird das entgegen gesetzte Ende des Bowdenzugs heraus gedrückt und die lineare Bewegung des Bowdenzugendes wird zum Beispiel über einen an der zugehörigen Branche angebrachten Hebel in eine Drehbewegung der Branche übertragen. Genauer gesagt wird die entsprechende Branche so gedreht, dass das jeweilige Maul geöffnet wird, wenn der Griff betätigt, das heißt der Bügel zusammengedrückt wird. Wenn der Griff freigegeben wird, bewegt sich das Ende des Bügels, an dem der Bowdenzug befestigt ist, durch die Elastizität des Bügels von dem an dem Schaftende montierten Ende des Bügels weg. Dadurch wird der Bowdenzug an dem hinteren Ende des Schafts ein Stück weit aus dem Schaft heraus gezogen und an dem entgegen gesetzten Ende (dem vorderen Ende) des Schafts ein Stück weit in den Schaft hinein gezogen. Durch diesen Vorgang wird die zugehörige Branche so gedreht, dass das entsprechende Maul geschlossen wird. Die Bowdenzüge beeinflussen sich gegenseitig in dem Schaft nicht nachteilig. Allgemein beeinflussen sich die Steuermechanismen nicht nachteilig. Es ist auch möglich, die Griffelemente so zu gestalten, dass das Öffnen der Mäuler der Greifgeräte durch die Elastizität der Griffelemente bewirkt wird und das jeweilige Griffelement betätigt wird, um das entsprechende Maul zu schließen. Die Steuervorrichtungen können aber auch so ausgebildet sein, dass kein Element vorgesehen ist, das die zugehörige Branche in ihre Ausgangsposition zurückführt, wenn die Steuervorrichtung freigegeben wird. Das heißt, das jeweilige Griffelement verbleibt in der zuletzt vom Nutzer eingestellten Position. Zudem kann beispielsweise eine Rasteinrichtung vorgesehen sein, sodass sich die eingestellte Position nicht versehentlich verändert.

Die Griffelemente können an einem gemeinsamen Griff vorgesehen sein. Die Betätigung der jeweiligen Branche kann dann zum Beispiel durch ein Drehen der Steuervorrichtung um die Achse des Greifgeräts oder um eine andere Achse durch eine Betätigung eines Druckknopfes etc. vorgenommen werden. Die Branchen können mechanisch, elektrisch, magnetisch, hydraulisch oder pneumatisch mit den Steuervorrichtungen verbunden sein. Die Steuervorrichtungen können so aufgebaut sein, dass die Branchen normalerweise geöffnet oder normalerweise geschlossen sind. Es ist auch möglich, dass für mehrere Branchen nur eine Steuervorrichtung vorgesehen ist, wobei bei der Betätigung der Steuervorrichtung zunächst eine erste Branche betätigt wird und anschließend eine weitere Branche betätigt wird. Hierbei ist es vorteilhaft, wenn die Branchen so unterscheidbar sind, dass die Reihenfolge der Betätigung der mindestens zwei Branchen vorhersehbar ist.

Im Gegensatz dazu können die Griffelemente aber auch einzeln oder gruppenweise an verschiedenen Griffen vorgesehen sein. Dies ist insbesondere sinnvoll, wenn drei oder mehr Branchen an dem Greifgerät vorhanden sind. Dann sind mehrere Personen erforderlich, wenn die Branchen gleichzeitig betätigt werden sollen. Dies ist einfacher zu handhaben, wenn die Griffelemente an mehreren Griffen angebracht sind, so dass die Griffelemente für den entsprechenden Nutzer leicht zugänglich sind. Hierzu können die zugehörigen Übertragungselemente über das hintere Ende des gemeinsamen Schafts hinaus verlängert sein und in einzelnen Schäften verlaufen.

Gemäß einer weiteren Ausführungsform des medizinischen Greifgeräts sind die Branchen und/oder die zugehörigen Stegelemente unterscheidbar. Außerdem sind die zugehörigen Steuervorrichtungen unterscheidbar, wobei eine eindeutige Zuordnung der entsprechenden Branche zu der zugehörigen Steuervorrichtung möglich ist. Auf diese Weise besteht vor einer Betätigung einer Steuervorrichtung Gewissheit darüber, welche Branche betätigt wird. Zudem kann auf diese Weise sicher entschieden werden, welche Steuervorrichtung betätigt werden soll, um ein bestimmtes Maul zu öffnen. Eine Betätigung der falschen Branche kann dazu führen, dass ein bereits gegriffenes Gewebestück verloren geht und dadurch die Gesamtzeit der Operation erheblich verlängert wird. Es kann auch beispielsweise zur Folge haben, dass die Randabschnitte durch das häufige manipulieren so ausfranst, sodass der Verschluss der Öffnung nicht mehr sicher durchgeführt werden kann. In diesem Fall muss oft ein offner Zugriff mit all seinen nachteiligen Folgen vorgenommen werden.

Insbesondere sind die Branchen und/oder Stegelemente durch ihre Farbe, ihre Form und/oder ihr Material unterscheidbar. So können zum Beispiel Symbole oder Nuten an den Branchen und/oder den Stegelementen ausgebildet sein, die sich entsprechend an den zugehörigen Steuervorrichtungen wieder finden. Es können auch Gravuren vorgesehen sein. Farben eignen sich zur Unterscheidung nur dann, wenn die Branchen und/oder Stegelemente mit einer Farbkamera betrachtet werden können. Das Material kann zur Unterscheidung hergezogen werden, wenn es zum Beispiel eine unterschiedliche Oberflächenbeschaffenheit besitzt, wie zum Beispiel eine unterschiedliche Rauheit, eine unterschiedliche Reflexion oder eine unterschiedliche Farbe. Eine Unterschiedliche Durchsichtigkeit des Materials kann auch zur Unterscheidung herangezogen werden. Es ist wahrscheinlich, dass zukünftig vermehrt Kunststoffe für medizinische Zwecke verwendet werden. Diese können unter Umständen transparent sein.

Gemäß einer weiteren vorteilhaften Ausbildung des medizinischen Greifgeräts weisen die Branchen und/oder die Stegelemente Schnittkanten auf. Mit diesen Schnittkanten können zum Beispiel Gewebeteile durchtrennt werden oder es kann eine Naht aufgetrennt werden.

Vorteilhafter Weise sind die Branchen und die Stegelemente symmetrisch angeordnet und die Branchen sind in radialer Richtung zu der Achse des Greifgeräts drehbar. Das heißt, dass die Branchen und die Stegelemente in Umfangsrichtung des Greifgeräts gleichmäßig verteilt angeordnet sind.

Gemäß einer weiteren vorteilhaften Ausbildung des medizinischen Greifgeräts sind die Stegelemente in radialer Richtung zu der Achse des Greifgeräts drehbar. Alternativ dazu können sich die Branchen in tangentialer Richtung zu der Achse des Greifgeräts oder in einer anderen Richtung zu dieser bewegen.

Gemäß einer weiteren vorteilhaften Ausbildung des medizinischen Greifgeräts der Steg und die Branchen zu ihrem vorderen Ende hin gleich lang. Sie können aber auch so ausgebildet sein, dass der Steg zum Beispiel weiter nach vorne vorsteht als die Branchen. Die einzelnen Stegelemente und die einzelnen Branchen können aber auch untereinander unterschiedlich lang ausgebildet sein.

### Vorteilhafte Wirkungen der Erfindung

### Kurze Beschreibung der Abbildungen der Zeichnungen

Diese und weitere Aufgaben, Merkmale und Vorteile der vorliegenden Erfindung sind aus den Ausführungsbeispielen ersichtlich, die im Folgenden unter Bezugnahme auf die beigefügten Figuren detailliert beschrieben sind.

Fig. 1 ist eine seitliche Ansicht eines Kopfs eines medizinischen Greifgeräts gemäß einem ersten Ausführungsbeispiel der vorliegenden Erfindung, dem beide Mäuler geöffnet sind.

Fig. 2 ist eine seitliche Ansicht des Kopfs des medizinischen Greifgeräts gemäß dem ersten Ausführungsbeispiel, bei dem ein Maul geöffnet ist.

Fig. 3 ist eine seitliche Ansicht eines Griffs des medizinischen Greifgeräts gemäß dem ersten Ausführungsbeispiel.

Fig. 4A, 4B und 4C sind schematische Darstellungen eines Kopfs eines medizinischen Greifgeräts gemäß einem fünften Ausführungsbeispiel in verschiedenen Zuständen.

Fig. 5A, 5B und 5C sind schematische Darstellungen eines Kopfs eines medizinischen Greifgeräts gemäß einem sechsten Ausführungsbeispiel in verschiedenen Zuständen.

Fig. 6A, 6B und 6C sind schematische Darstellungen von Griffen eines medizinischen Greifgeräts gemäß verschiedenen Ausführungsbeispielen.

Fig. 7A und 7B sind seitliche Ansichten eines medizinischen Greifgeräts gemäß einem zweiten Ausführungsbeispiel der vorliegenden Erfindung, wobei in der Fig. 7A die Mäuler geöffnet und die Stegelemente beabstandet sind und in der Fig. 7B die Mäuler geschlossen sind und die Stegelemente zusammengedrückt sind.

### Bester Weg zur Ausführung der Erfindung

### Weg(e) zur Ausführung der Erfindung

(erstes Ausführungsbeispiel)

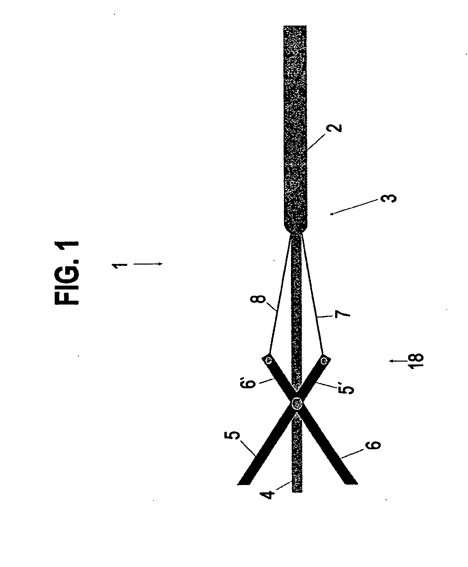

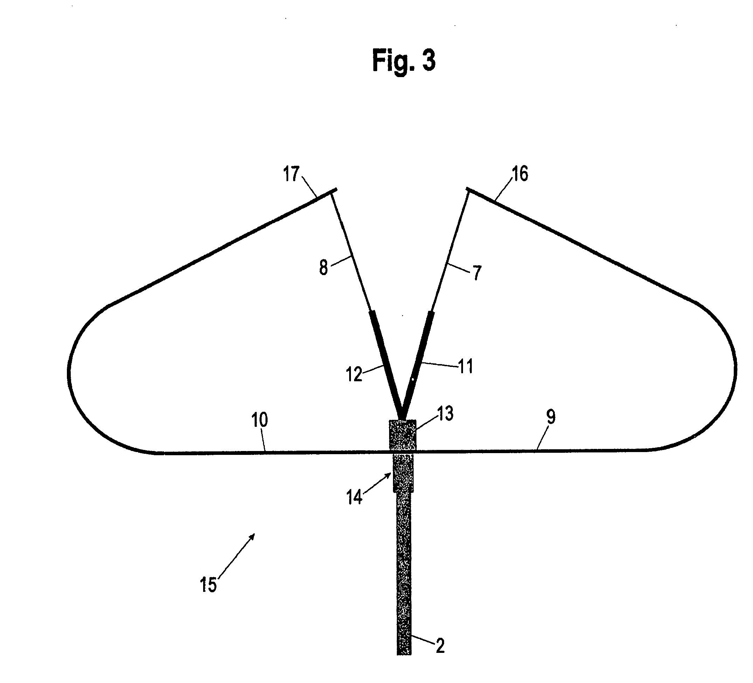

Im Folgenden ist ein erstes Ausführungsbeispiel des medizinischen Greifgeräts der vorliegenden Erfindung unter Bezugnahme auf die Fig. 1 bis 3 beschrieben.

Das medizinische Greifgerät (1) des ersten Ausführungsbeispiels besitzt einen flexiblen Schaft (2) mit einem vorderen Ende (3) und einem hinteren Ende (14). Der Schaft (2) ist als eine druckfeste biegeweiche Spirale ausgebildet. Zudem besitzt es einen Steg (4) bestehend aus einem einzigen Stegelement, das an dem vorderen Ende (3) des Schafts (2) befestigt ist. Zwei Branchen (5, 6) sind an dem Steg (4) drehbar angelenkt. Teile von zwei flexiblen Steuermechanismen (7, 9, 8, 10) sind in dem Schaft (2) angeordnet und jede einzelne Branche (5, 6) ist mittels eines eigenen Steuermechanismus (7, 9, 8, 10) gegenüber dem Steg (4) bewegbar. In diesem Fall ist die in der Fig. 1 linke Branche (6) durch den Steuermechanismus (8, 10) steuerbar und die in der Fig. 1 rechte Branche (5) ist durch den Steuermechanismus (7, 9) steuerbar. Bei dem ersten Ausführungsbeispiel des medizinischen Greifgeräts besitzen die Steuermechanismen (7, 9, 8, 10) jeweils ein zug- und druckfestes Übertragungselement (7, 8), die gemeinsam in dem Schaft (2) verlaufen. Jedes dieser Übertragungselemente (7, 8) ist an dem vorderen Ende (3) des Schafts (2) mit einer Branche (5, 6) verbunden. Zudem sitzen die Steuermechanismen (7, 9, 8, 10) jeweils eine Steuervorrichtung (9, 10), die an dem hinteren Ende (14) des Schafts (2) vorgesehen und mit dem zugehörigen Übertragungselement (7, 8) verbunden sind, wobei

jedes Übertragungselement (7, 8) die von der entsprechenden Steuervorrichtung (9, 10) ausgegebene Bewegung an die entsprechende Branche (5, 6) überträgt.

Bei diesem Ausführungsbeispiel sind die Übertragungselemente (7, 8) Bowdenzüge und die Steuervorrichtungen (9, 10) sind Griffelemente. Die Griffelemente (9, 10) werden betätigt, um die entsprechende Branche (5, 6) zu öffnen, und sie werden freigegeben bzw. losgelassen, um die entsprechende Branche (5, 6) zu schließen. Die Griffelemente (9, 10) sind an einem gemeinsamen Griff (15) vorgesehen.

Die Branchen (5, 6) und/oder das zugehörige Stegelement (4) sind bei diesem Ausführungsbeispiel nicht unterscheidbar. Allerdings können bei einem medizinischen Greifgerät (1) gemäß diesem Ausführungsbeispiel auch noch nachträglich Nuten oder Gravuren an den Branchen (5, 6) und/oder dem Stegelement (4) und den zugehörigen Griffelementen (9, 10) vorgesehen werden, so dass dann eine eindeutige Zuordnung von Griffelement (9, 10) zu Branche (5, 6) möglich ist. Da bei diesem Ausführungsbeispiel nur ein Stegelement (4) vorgesehen ist, kann die Gravur oder Nut zur Unterscheidung beispielsweise an beiden Seitenflächen des Stegelements (4) vorgesehen sein, wobei zum Beispiel eine Nut an beiden Seitenflächen des Stegelements (4) so vorgesehen wird, dass sie näher zu der Branche (5) ist, und das zu der Branche (5) zugehörige Griffelement (9) ebenfalls mit einer Nut versehen wird.

Die Branchen (5, 6) und das eine Stegelement (4) sind symmetrisch angeordnet. Das heißt, die Längsachse des eines Stegelements (4) ist mit der Längsachse des medizinischen Greifgeräts (1) identisch und die beiden Branchen (5, 6) sind diametral gegenüberliegend an dem Stegelement (4) angelenkt. Die Branchen (5, 6) sind zudem in radialer Richtung zu der Achse des Greifgeräts (1) drehbar.

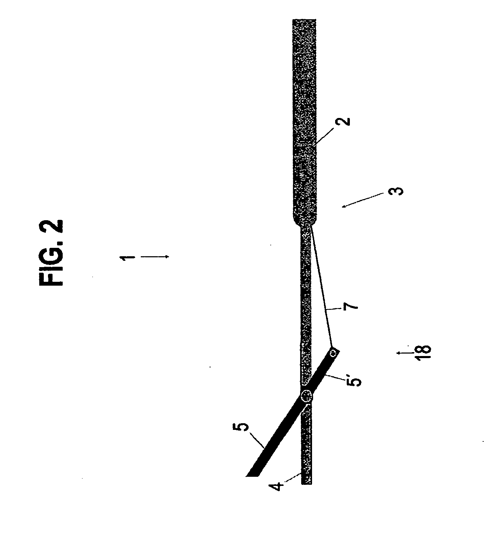

Wie dies in den Fig. 1 und 2 gezeigt ist, sind die Bowdenzüge (7, 8) an Fortsätzen bzw. Hebeln (5', 6') der Branchen (5, 6) befestigt. Diese Fortsätze bzw. Hebel (5', 6') sind so ausgebildet, dass sie sich in einer Aussparung des Stegelements (4) befinden, wenn die Branchen (5, 6) geschlossen sind. Dadurch kann der Durchmesser des Kopfs (18) des Greifgeräts (1) im geschlossenen Zustand sehr klein gehalten werden. Der Kopf (18) des Greifgeräts (1) bezeichnet die Elemente, die an dem vorderen Ende (3) des Greifgeräts (1) vorgesehen sind.

Die Bowdenzüge (7, 8) sind mittels Gelenkmechanismen an den Fortsätzen (5', 6') der Branchen (5, 6) befestigt. Das vordere Ende des Stegelements (4) ist etwas verdickt und die vorderen Enden der Branchen (5, 6) sind leicht zu dem Stegelement (4) hin so gebogen und verjüngt, dass sie in das vordere Ende des Stegelements (4) eingreifen, in dem eine entsprechende Vertiefung vorgesehen ist. Die beiden Branchen (5, 6) sind gleich groß und an derselben Achse an dem Stegelement (4) angelenkt.

An dem hinteren Ende (14) des Schafts (2) ist bei diesem Ausführungsbeispiel ein Griff (15) ausgebildet, der im Wesentlichen aus den Griffelementen (9, 10) besteht. Die Griffelemente (9, 10) sind auf eine Verdickung aufgeschoben, die an dem hinteren Ende (14) des Schafts (2) vorgesehen ist, und mittels eines Innengewindeelements (13) an dem Schaft (2) fixiert. Aus dem Gewindeelement (13) ragen die beiden Bowdenzüge (7, 8) hervor. Die Hülsen (11, 12) dienen lediglich der Führung der Bowdenzüge (7, 8). Die Griffelemente (9, 10) sind als elastische U-förmige Bügel aus Metall ausgebildet. An den freien Enden (16, 17) der Bügel (9, 10) sind die Bowdenzüge (7, 8) befestigt, wobei der letzte Abschnitt eines jeden Bowdenzugs (7, 8) steif ausgebildet ist und bei einer Betätigung des Griffelements (9, 10) in die jeweilige Hülse (11, 12) eingeführt wird. Bei diesem Ausführungsbeispiel sind die Mäuler des Greifgeräts (1) im Ausgangszustand geschlossen und werden durch eine Betätigung der Griffelemente (9, 10) geöffnet.

(zweites Ausführungsbeispiel)

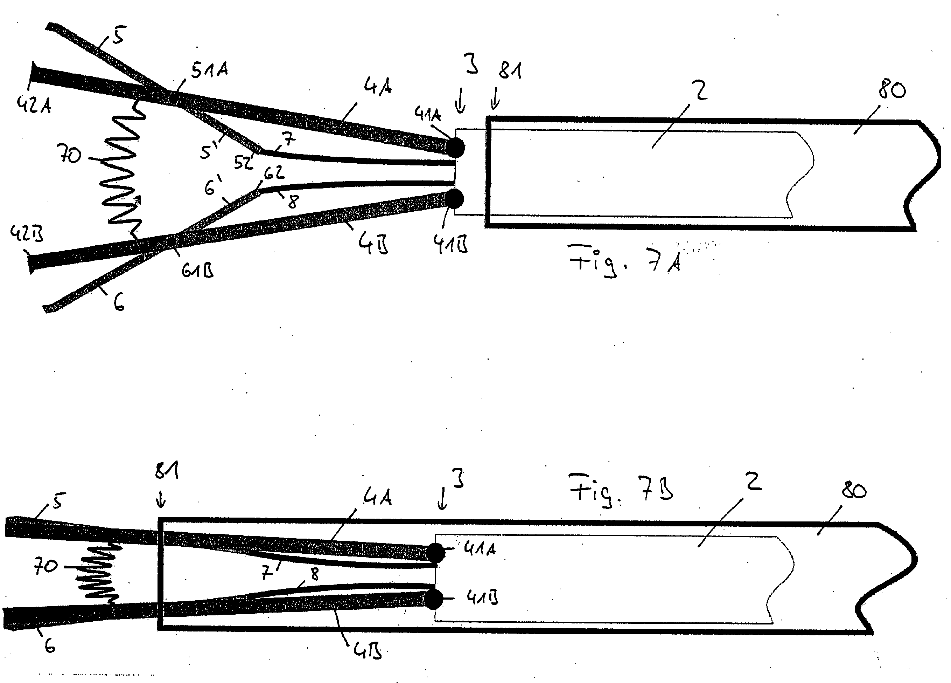

Ein zweites Ausführungsbeispiel des medizinischen Greifgeräts ist unter Bezugnahme auf die Fig. 7A und 7B beschrieben.

Das medizinische Greifgerät (1) des zweiten Ausführungsbeispiels besitzt auch einen flexiblen Schaft (2) mit einem vorderen Ende (3) und einem hinteren Ende (14), einen Steg (4) bestehend aus zwei Stegelementen (4A, 4B), wobei die Stegelemente (4A, 4B) an dem vorderen Ende (3) des Schafts (2) befestigt sind, zwei Branchen (5, 6), die an den Stegelementen (4A, 4B) an den Anlenkpunkten (51A, 61 B) angelenkt sind, und zwei flexible Steuermechanismen (7, 9, 8, 10), die zumindest teilweise in dem Schaft (2) angeordnet sind. Die Branche (5) ist durch den Steuermechanismus (7, 9) gegenüber dem Stegelement (4A) drehbar und die Branche (6) ist durch den Steuermechanismus (8, 10) gegenüber dem Stegelement (4B) drehbar. Bei diesem Ausführungsbeispiel besteht der Steg (4) aus zwei Stegelementen (4A, 4B), wobei keines der Stegelemente (4A, 4B) starr an dem Schaft befestigt ist. Ein Element (70) ist an den Stegelementen (4A, 4B) so angeordnet ist, dass die Stegelemente (4A, 4B) relativ zueinander um die Anlenkpunkte (41A, 41 B) herum gedreht werden können. Das Element (70) ist ein elastisches Element, das heißt in diesem Fall eine Druckfeder. Die Druckfeder (70) drängt die körperfernen Enden (42A, 42B) der Stegelemente (4A, 4B) auseinander. Wenn allerdings, wie dies in der Fig. 8B gezeigt ist, das Greifgerät (1) ein Stück weit in einen Arbeitskanal (80) eines Endoskops hinein gezogen wird, werden die Stegelemente (4A, 4B) durch die Bewegung und die Anlage an dem Rand (81) des Arbeitskanals (80) aufeinander zu gedrückt und die Druckfeder (70) wird auch zusammen gedrückt. Wie bei dem ersten Ausführungsbeispiel besitzen die Steuermechanismen (7, 9, 8, 10) jeweils ein zug- und druckfestes Übertragungselement (7, 8), die gemeinsam in dem Schaft (2) verlaufen, wobei jedes dieser Übertragungselemente (7, 8) an dem vorderen Ende (3) des Schafts (2) mit einer Branche (5, 6) verbunden ist. Genauer gesagt ist das Übertragungselement (7) an dem Befestigungspunkt (52) an dem Hebel (5') der Branche (5) gelenkig befestigt und das Übertragungselement (8) an dem Befestigungspunkt (62) an dem Hebel (6') der Branche (6) gelenkig befestigt. Jedes Übertragungselement (7, 8) überträgt die von der entsprechenden Steuervorrichtung (9, 10) ausgegebene Bewegung an die entsprechende Branche (5, 6).

Bei dem zweiten Ausführungsbeispiel sind die Übertragungselemente (7, 8) Bowdenzüge. Die Griffelemente (9, 10) sind in Form und Funktion bis auf die Tatsache identisch zu den Griffelementen (9, 10) des ersten Ausführungsbeispiels, dass das Griffelement (9) eine Nut aufweist. Auch bei dem zweiten Ausführungsbeispiel sind die Griffelemente (9, 10) an einem Griff (15) vorgesehen.

Die Branchen (5, 6) sind unterscheidbar, wobei die Branche (5) an ihrer Außenseite eine ähnliche Nut wie das Griffelement (9) aufweist. Somit ist für den Nutzer erkennbar, welches Griffelement (9, 10) welche Branche (5, 6) steuert.

Wie dies in den Fig. 7A und 7B gezeigt ist, sind die Branchen (5, 6) und die Stegelemente (4A, 4B) symmetrisch angeordnet und die Branchen (5, 6) sind in radialer Richtung zu der Achse des Greifgeräts (1) um die Anlenkpunkte (51A, 61 B) herum drehbar. Die Stegelemente (4A, 4B) sind um die Anlenkpunkte (41A, 41 B) herum in radialer Richtung zu der Achse des Greifgeräts (1) drehbar.

Mit dem Greifgerät (1) des zweiten Ausführungsbeispiels können dieselben Effekte wie mit dem Greifgerät (1) des ersten Ausführungsbeispiels erzielt werden. Zudem kann durch die Ausbildung des Stegs (4) aus zwei Stegelementen (4A, 4B) der Abstand der beiden Mäuler des Greifgeräts (1) eingestellt werden, wenn das Greifgerät (1) zusammen mit einem Endoskop verwendet wird. Dann kann der Kopf (18) des Greifgeräts (1) des zweiten Ausführungsbeispiels so weit in den Arbeitskanal (80) des Endoskops hinein gezogen werden, dass die Stegelemente (4A, 4B) an dem vorderen Rand (81) des Arbeitskanals (80) des Endoskops anliegen und durch diesen Rand (81) zusammen gedrückt werden. Das Hineinziehen des Kopfs (18) des Greifgeräts (1) wird dadurch erreicht, dass man an dem anderen Ende des Arbeitskanals (80) an dem Schaft (2) des Greifgeräts (1) zieht.

(drittes Ausführungsbeispiel)

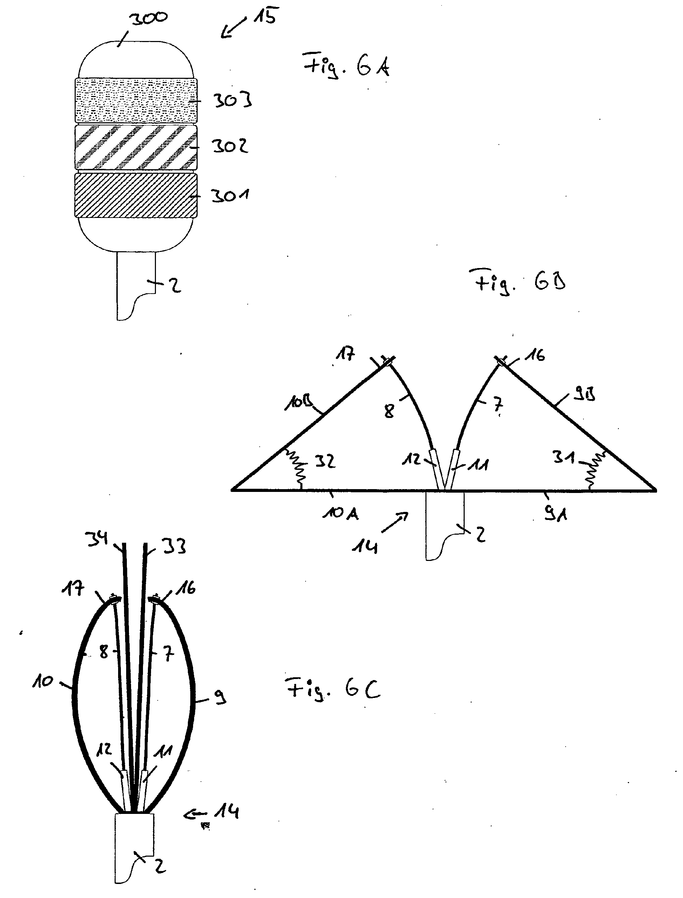

Im Folgenden ist ein drittes Ausführungsbeispiel des Greifgeräts unter Bezugnahme auf die Fig. 6B beschrieben. Das dritte Ausführungsbeispiel unterscheidet sich von dem ersten Ausführungsbeispiel nur durch den Aufbau des Griffs (15). Der Griff (15) ist an dem hinteren Ende (14) des Schafts (2) angebracht. Die Griffelemente (9, 10) bestehen jeweils aus zwei Teilen (9A, 9B, 10A, 10B), wobei die Teile (9A, 10A) an dem hinteren Ende (14) des Schafts (2) befestigt sind. Die jeweils zwei Griffelementteile (9A, 9B) und (10A, 10B) sind gelenkig verbunden. Zwischen den Griffelementteilen (9A, 9B) und (10A, 10B) ist jeweils eine Druckfeder (31, 32) vorgesehen. Der restliche Aufbau und die Funktion der Griffs (15) des dritten Ausführungsbeispiels entsprechen dem ersten Ausführungsbeispiel und werden daher nicht erneut erklärt.

(viertes Ausführungsbeispiel)

Ein viertes Ausführungsbeispiel des erfindungsgemäßen Greifgeräts ist unter Bezugnahme auf die Fig. 6C beschrieben. Das vierte Ausführungsbeispiel unterscheidet sich von dem ersten Ausführungsbeispiel im Aufbau und der Funktion des Griffs (15). Die elastischen Bügel (9, 10) des ersten Ausführungsbeispiels sind hier nicht U-förmig gebogen sondern nehmen eher eine C-Form an. Die Bügel werden bei einer Betätigung aber anders als bei dem ersten Ausführungsbeispiel verformt. Bei einer Betätigung der Griffelemente (9, 10) bewegen sich die hinteren Enden (16, 17) der Bügel (9, 10) von dem Schaft (2) weg und die Bowdenzüge (7, 8) werden ein Stück weit aus dem Schaft (2) herausgezogen. Dies ist daher möglich, da bei diesem vierten Ausführungsbeispiel die Branchen (5, 6) im Gegensatz zu dem ersten Ausführungsbeispiel im Ausgangszustand geöffnet sind und bei einer Betätigung des entsprechenden Griffelements (9, 10) geschlossen werden. Zwischen den Bügeln (9, 10) sind Abstandselemente (33, 34) vorgesehen. Diese Abstandselemente (33, 34) verhindern, dass bei einer Betätigung eines der Griffelemente (9, 10) das hintere Ende (16, 17) des betätigten Griffelements (9, 10) in Kontakt mit dem anderen Griffelement (9, 10) oder dem zugehörigen Bowdenzug (7, 8) kommt und somit den Zustand oder die Betätigung der anderen Branche (5, 6) beeinflusst. Bei dieser Form der Griffelemente (9, 10) ist es aber in der Praxis vorteilhaft, an einem Griff (15) nur jeweils ein Griffelement (9, 10) vorzusehen.

(fünftes Ausführungsbeispiel)

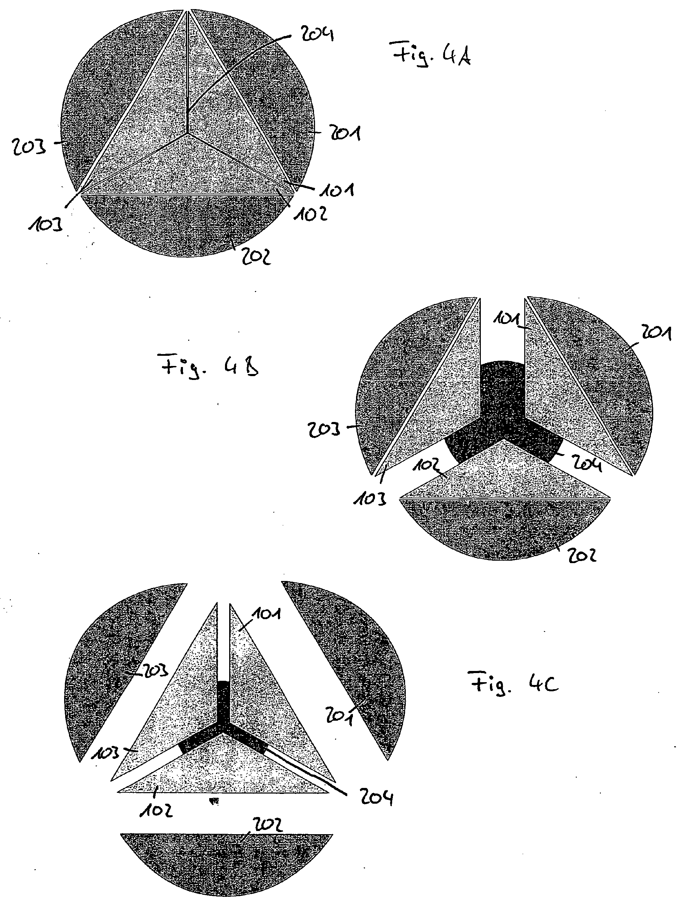

Ein fünftes Ausführungsbeispiel des Greifgeräts (1) ist unter Bezugnahme auf die Fig. 4A bis 4C und 6A beschrieben. Bei diesem fünften Ausführungsbeispiel besitzt das Greifgerät (1) drei Stegelemente (101, 102, 103) und drei Branchen (201, 202, 203). Dementsprechend besitzt der Griff (15) des Greifgeräts (1) drei Steuervorrichtungen (301, 302, 303), die den Branchen (201, 202, 203) eindeutig zuordenbar sind. Die Zuordnung erfolgt bei diesem Ausführungsbeispiel über Gravuren an den Umfangsflächen der Branchen (201, 202, 203) und der Stegelemente (101, 102, 103). Die Fig. 4A, 4B und 4C zeigen jeweils einen Schnitt senkrecht zu der Längsachse des Greifgeräts (1) durch den Kopf (18) des Greifgeräts (1). In der Fig. 4A liegen die Stegelemente (101, 102, 103) aneinander an und die Mäuler sind alle geschlossen. Dieser Zustand entspricht beispielsweise dem hindurchführen des Greifgeräts (1) durch einen Arbeitskanal eines Endoskops. In der Fig. 4B sind die Mäuler des Greifgeräts (1) auch geschlossen, aber die Stegelemente (201, 202, 203) sind zueinander beabstandet. Die Stegelemente (101, 102, 103) werden bei diesem Ausführungsbeispiel durch einen Gummiball (204) auseinander gedrückt, der zwischen den Stegelementen (101, 102, 103) in dafür vorgesehenen Vertiefungen gehalten wird. Im der Fig. 4C sind die Stegelemente (101, 102, 103) weniger zueinander beabstandet als in der Fig. 4B. Das heißt, die Stegelemente (101, 102, 103) liegen an dem Rand des Arbeitskanals des Endoskops an und der Kopf (18) des Greifgeräts (1) ist ein Stück weit in den Arbeitskanal hineingezogen worden. Die Branchen (201, 202, 203) sind alle geöffnet und somit bereit zu greifen.

Der Griff (15) des Greifgeräts (1) des fünften Ausführungsbeispiels besitzt einen Grundkörper (300), an dem Steuervorrichtungen (301, 302, 303) angebracht sind. Die Steuervorrichtungen (301, 302, 303) sind als Drehelemente ausgebildet, die an ihrer Umfangsseite die Gravur aufweisen, die der Gravur der jeweils zugehörigen Branche (201, 202, 203) entspricht. Um eine Branche (201, 202, 203) zu bewegen, wird das entsprechende Drehelement (301, 302, 303) um den Grundkörper (300) gedreht. Im Inneren des Grundkörpers (300) sind Mechanismen angeordnet, die eine Drehbewegung eines jeden Drehelements (301, 302, 303) in eine translatorische Bewegung des zugehörigen Bowdenzugs umsetzt. An den Drehelementen (301, 302, 303) können im inneren des Grundkörpers (300) zudem elastische Elemente angeordnet sein, die das jeweilige Drehelement (301, 302, 303) wieder selbsttätig in die Ausgangsposition zurückführen, wenn das Drehelement (301, 302, 303) freigegeben wird. Eine Bewegung eines Drehelements (301, 302, 303) nach rechts in der Ansicht der Fig. 6A kann zum Beispiel die jeweilige Branche (201, 202, 203) öffnen, und eine entgegen gesetzte Bewegung kann die jeweilige Branche (201, 202, 203) schließen. Mit dieser Art von Griff (15) ist es einfach, mehr als zwei Steuervorrichtungen an einem Griff (15) so vorzusehen, dass sie leicht betätigt werden können. Sieht man an den Drehelementen (301, 302, 303) zudem noch Elemente vor, die eine selbsttätige Bewegung der Drehelemente (301, 302, 303) verhindern, so kann das Greifgerät (1) leicht durch eine einzelne Person gesteuert werden.

(sechstes Ausführungsbeispiel)

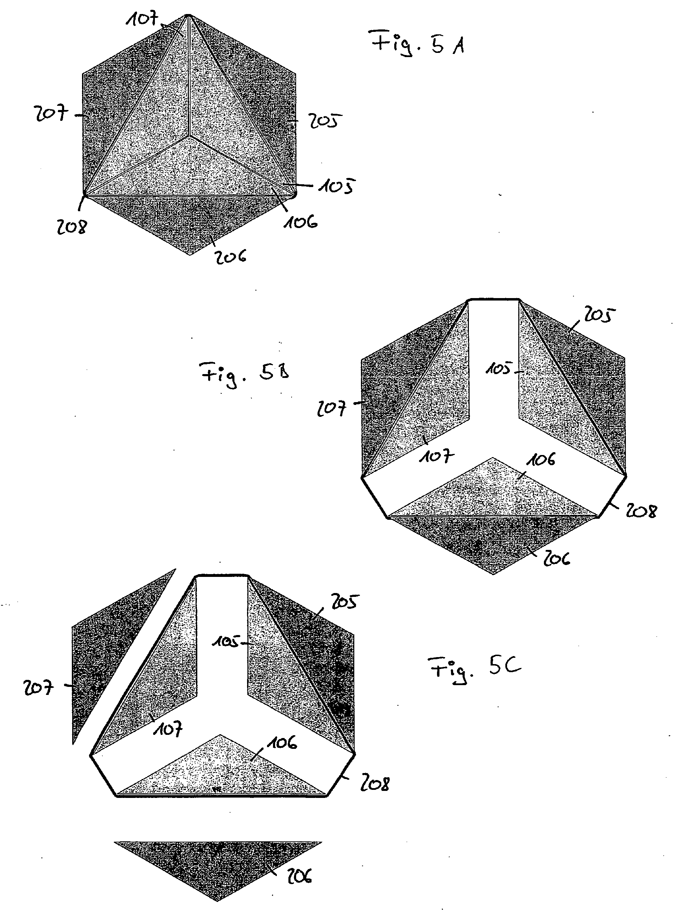

Bei dem sechsten Ausführungsbeispiel des Greifgeräts (1), das in den Fig. 5A bis 5C gezeigt ist, unterscheidet sich die Form der Branchen (205, 206, 207) von dem fünften Ausführungsbeispiel. Zudem werden bei diesem Ausführungsbeispiel die Stegelemente (105, 106, 107) durch ein Gummiband (208) zusammen gezogen. Bei diesem Ausführungsbeispiel sind die Stegelemente (105, 106, 107) so ausgebildet, dass sie elektromagnetische Einrichtungen besitzen, die die Stegelemente (105, 106, 107) auseinander drängen, wenn sie aktiviert sind. Die Stärke der magnetischen Abstoßung lässt sich über den zugeführten Strom einstellen. Das Gummiband (208) besitzt eine vorgegebene Elastizität. Daher kann über die eingestellte magnetische Abstoßung der Stegelemente (105, 106, 107) der Abstand der Stegelemente zueinander eingestellt werden. Die Übertragungselemente für die Branchen (205, 206, 207) dieses Ausführungsbeispiels sind wie bei den vorangehenden Ausführungsbeispielen Bowdenzüge. Die elektrischen Leitungen für die elektromagnetischen Einrichtungen sind bei diesem Ausführungsbeispiel in dem Mantel des Schafts (2) des Greifgeräts (1) untergebracht. Der Griff (15) des Greifgeräts (1) dieses Ausführungsbeispiels verfügt daher gegenüber dem in der Fig. 6A dargestellten Griff (15) über noch ein weiteres Drehelement, mit dessen Hilfe der elektrische Strom für die elektromagnetischen Einrichtungen eingestellt werden kann.

(weitere Ausführungsbeispiele)

Gemäß einem weiteren Ausführungsbeispiel können die Branchen (205, 206, 207) des sechsten Ausführungsbeispiels auch mittels Miromotoren betätigt werden. Dann kann der Griff (15) zum Beispiel so ausgebildet sein, dass er vier Drehelemente besitzt, von denen drei Drehelemente die Öffnung der jeweiligen Branche (205, 206, 207) steuern und ein Drehelement den Abstand der Stegelemente (105, 106, 107) steuert. Die Drehelemente sind dann um Beispiel als Drehpotentiometer ausgebildet. Alternativ dazu können die Zustände auch mit Hilfe eins Computers gesteuert werden. Als weitere Alternative Steuervorrichtungen bieten sich elektrische Schieberegler an.

Gemäß einem weiteren Ausführungsbeispiel kann aber auch der Abstand der Stegelemente zueinander mittels eines Bowdenzugs eingestellt werden.

Gemäß einem weiteren Ausführungsbeispiel der Erfindung sind die Bowdenzüge so ausgebildet, dass die Bowdenzugseile an den Griffelementen ein Stück weit länger als die Bowdenzugummantelungen sind. An dem Ende der Bowdenzüge sind Abschlusselemente in der Form von Kreisscheiben angebracht. Die hinteren Enden der Bowdenzugummantelungen sind mit Ringelementen versehen, wobei die Bowdenzüge durch die Ringelemente hindurch geführt sind. Zwischen den Ringelementen der Bowdenzugummantelungen und den Abschlusselementen der Bowdenzugseile sind jeweils Spiraldruckfedern so angeordnet, dass die Bowdenzugseile entlang der Achsen der Spiralfedern verlaufen und die Spiralfedern jeweils an einem Abschlusselement und einem Ringelement anliegen und diese auseinander drücken. Wenn nun das Abschlusselement durch den Nutzer zu dem Ringelement hin gedrückt wird, wird die dazwischen befindliche Spiralfeder gedrückt und die zugehörige Branche öffnet sich. Wenn das Abschlusselement freigegeben wird, führt die Spiralfeder das Abschlusselement in seine Ausgangsposition zurück und schließt die jeweilige Branche.

Das Greifgerät (1), das in dieser Beschreibung nur für medizinische Zwecke beschrieben ist, kann beispielsweise auch in der Feinmechanik eingesetzt werden.

Es ist das medizinisches Greifgerät (1) mit dem flexiblen Schaft (2), der das vordere Ende (13) und das hintere Ende (14) besitzt, dem Steg (4) bestehend aus mindestens einem Stegelement, wobei der Steg (4) an dem vorderen Ende (3) des Schafts (2) befestigt ist, den mindestens zwei Branchen (5, 6), die an dem Steg (4) angelenkt sind, und den mindestens zwei flexiblen Steuermechanismen (7, 9, 8,10) offenbart, die zumindest teilweise in dem Schaft (2) angeordnet sind. Bei dem erfindungsgemäßen medizinischen Greifgerät (1) ist jede einzelne Branche (5, 6) mittels eines eigenen Steuermechanismus (7, 9, 8, 10) gegenüber dem Steg (4) individuell bewegbar.

## Patentansprüche

1. Medizinisches Greifgerät (1) mit einem Schaft (2) mit einem vorderen Ende (13) und einem hinteren Ende (14),
einem Steg (4) bestehend aus mindestens einem Stegelement, wobei der Steg (4) an dem vorderen Ende (3) des Schafts (2) befestigt ist,
mindestens zwei Branchen (5, 6), die an dem Steg (4) angelenkt sind, und mindestens zwei flexiblen Steuermechanismen (7, 9, 8, 10), die zumindest teilweise in dem Schaft (2) angeordnet sind, wobei jede einzelne Branche (5, 6) mittels eines eigenen Steuermechanismus (7, 9, 8, 10) gegenüber dem Steg (4) individuell bewegbar ist, um zumindest zwei Mäuler zu bilden, die unabhängig voneinander öffen- und schließbar sind, wobei
die Steuermechanismen (7, 9, 8, 10) jeweils ein zug- und druckfestes Übertragungselement (7, 8) besitzen, die gemeinsam in dem Schaft (2) verlaufen, wobei jedes dieser Übertragungselemente (7, 8) an dem vorderen Ende (3) des Schafts (2) mit einer Branche (5, 6) verbunden ist, und jeweils eine Steuervorrichtung (9, 10) besitzen, die an dem hinteren Ende (14) des Schafts (2) vorgesehen ist und mit dem zugehörigen Übertragungselement (7, 8) verbunden ist, wobei
jedes Übertragungselement (7, 8) die von der entsprechenden Steuervorrichtung (9, 10) ausgegebene Bewegung an die entsprechende Branche (5, 6) überträgt, **dadurch gekennzeichnet, dass** der Schaft (2) flexibel ist, dass
die Übertragungselemente (7, 8) Bowdenzüge sind, und dass die Steuervorrichtungen (9, 10) Griffelemente sind, wobei
die Griffelemente (9, 10) derart ausgestaltet sind, dass eine Betätigung der Griffelemente (9, 10) die entsprechende Branche (5, 6) öffnet und ein Freigeben der Griffelemente (9, 10) die entsprechende Branche (5, 6) schließt.

2. Medizinisches Greifgerät (1) gemäß Anspruch 1,
**dadurch gekennzeichnet, dass**
der Steg (4) aus mindestens zwei Stegelementen (4A, 4B) besteht, wobei maximal eines der Stegelemente (4A, 4B) starr an dem Schaft (2) befestigt ist und die restlichen Stegelemente (4A, 4B) schwenkbar an dem vorderen Ende des Schafts (2) oder dem maximal einen starren Stegelement (4A, 4B) angelenkt sind,
mindestens ein Betätigungselement (70) an den Stegelementen (4A, 4B) angeordnet ist, durch das die Stegelemente (4A, 4B) relativ zueinander bewegt werden können, wobei jeweils eine Branche (5, 6) an einem Stegelement (4A, 4B) drehbar angelenkt ist.

3. Medizinisches Greifgerät (1) gemäß Anspruch 2,
**dadurch gekennzeichnet, dass**
das Betätigungselement mindestens eine Feder (70) hat, die an den Stegelementen (4A, 4B) so angeordnet ist, dass sie die Stegelemente (4A, 4B) an ihrem körperfernen Ende (41 A, 41 B) auseinander drängt.

4. Medizinisches Greifgerät (1) gemäß Anspruch 1,
**dadurch gekennzeichnet, dass**
die Griffelemente (9, 10) an einem gemeinsamen Griff (15) vorgesehen sind.

5. Medizinisches Greifgerät (1) gemäß Anspruch 1,
**dadurch gekennzeichnet, dass**
die Griffelemente (9, 10) an einzelnen Griffen vorgesehen sind.

6. Medizinisches Greifgerät (1) gemäß einem der Ansprüche 1 bis 5,
**dadurch gekennzeichnet, dass**
die Branchen (5, 6) und/oder die zugehörigen Stegelemente (4A, 4B) unterscheidbar sind,
die zugehörigen Steuervorrichtungen (9, 10) unterscheidbar sind, sodass
eine eindeutige Zuordnung der entsprechenden Branche (5, 6) zu der zugehörigen Steuervorrichtung (9, 10) möglich ist.

7. Medizinisches Greifgerät (1) gemäß Anspruch 6,
**dadurch gekennzeichnet, dass**
die Branchen (5, 6) und/oder die Stegelemente (4A, 4B) durch ihre Farbe, ihre Form und/oder ihr Material unterscheidbar sind.

8. Medizinisches Greifgerät (1) gemäß einem der Ansprüche 1 bis 7,
**dadurch gekennzeichnet, dass**
die Branchen (5, 6) und/oder die Stegelemente (4A, 4B) Schnittkanten aufweisen.

9. Medizinisches Greifgerät (1) gemäß einem der Ansprüche 1 bis 8,
**dadurch gekennzeichnet, dass**
die Branchen (5, 6) und die Stegelemente (4A, 4B) symmetrisch angeordnet sind und die Branchen (5, 6) in radialer Richtung zu der Achse des Greifgeräts (1) drehbar sind.

10. Medizinisches Greifgerät (1) gemäß einem der Ansprüche 1 bis 9,
**dadurch gekennzeichnet, dass**
die Stegelemente (4A, 4B) in radialer Richtung zu der Achse des Greifgeräts (1) drehbar sind.

11. Medizinisches Greifgerät (1) gemäß einem der Ansprüche 1 bis 10,
**dadurch gekennzeichnet, dass**
der Steg (4) und die Branchen (5, 6) zu ihrem vorderen Ende hin gleich lang sind.

## Claims

1. Medical gripping device (1) having a shaft (2) having a front end (13) and a rear end (14),
a web (4) comprising at least one web element, the web (4) being secured to the front end (3) of the shaft (2),
at least two branches (5, 6) which are articulated to the web (4) and
at least two flexible control mechanisms (7, 9, 8, 10) which are at least partially arranged in the shaft (2), each individual branch (5, 6) being able to be individually moved with respect to the web (4) by means of a separate control mechanism (7, 9, 8, 10) in order to form at least two mouths which can be opened and closed independently of each other, the control mechanisms (7, 9, 8, 10) each having a tension and pressure-resistant transmission element (7, 8) which extend together in the shaft (2), each of these transmission elements (7, 8) being connected to the front end (3) of the shaft (2) by means of a branch (5, 6) and each having a control device (9, 10) which is provided at the rear end (14) of the shaft (2), and being connected to the associated transmission element (7, 8),
each transmission element (7, 8) transmitting the movement transmitted by the corresponding control device (9, 10) to the corresponding branch (5, 6),
**characterised in that** the shaft (2) is flexible, **in that** the transmission elements (7, 8) are Bowden cables, and **in that** the control devices (9, 10) are gripping elements, the gripping elements (9, 10) being constructed in such a manner that activation of the gripping elements (9, 10) opens the corresponding branch (5, 6) and release of the gripping elements (9, 10) closes the corresponding branch (5, 6).

2. Medical gripping device (1) according to claim 1,
**characterised in that**
the web (4) comprises at least two web elements (4A, 4B), a maximum of one of the web elements (4A, 4B) being rigidly secured to the shaft (2) and the remaining web elements (4A, 4B) being pivotably articulated to the front end of the shaft (2) or the maximum one rigid web element (4A, 4B),
at least one actuation element (70) being arranged on the web elements (4A, 4B) and the web elements (4A, 4B) being able to be moved thereby relative to each other, a branch (5, 6) being rotatably articulated to a web element (4A, 4B), respectively.

3. Medical gripping device (1) according to claim 2,
**characterised in that**
the actuation element has at least one spring (70) which is arranged on the web elements (4A, 4B) in such a manner that it urges the web elements (4A, 4B) apart at the end (41A, 41B) thereof remote from the body.

4. Medical gripping device (1) according to claim 1,
**characterised in that**
the gripping elements (9, 10) are provided on a common handle (15).

5. Medical gripping device (1) according to claim 1,
**characterised in that**
the gripping elements (9, 10) are provided on individual handles.

6. Medical gripping device (1) according to any one of claims 1 to 5,
**characterised in that**
the branches (5, 6) and/or the associated web elements (4A, 4B) can be differentiated,
the associated control devices (9, 10) can be differentiated so that
a clear association of the corresponding branch (5, 6) with the associated control device (9, 10) is possible, respectively.

7. Medical gripping device (1) according to claim 6,
**characterised in that**
the branches (5, 6) and/or the web elements (4A, 4B) can be differentiated by their colour, their shape and/or their material.

8. Medical gripping device (1) according to any one of claims 1 to 7,
**characterised in that**
the branches (5, 6) and/or the web elements (4A, 4B) have cutting edges.

9. Medical gripping device (1) according to any one of claims 1 to 8,
**characterised in that**
the branches (5, 6) and the web elements (4A, 4B) are arranged symmetrically and the branches (5, 6) can be rotated in a radial direction relative to the axis of the gripping device (1).

10. Medical gripping device (1) according to any one of claims 1 to 9,
**characterised in that**
the web elements (4A, 4B) can be rotated in a radial direction relative to the axis of the gripping device (1).

11. Medical gripping device (1) according to any one of claims 1 to 10,
**characterised in that**
the web (4) and the branches (5, 6) are of the same length in the direction towards the front end thereof.

## Revendications

1. Appareil médical de saisie (1) doté d'une hampe (2) comportant une extrémité avant (13) et une extrémité arrière (14),
une tige (4) consistant en au moins un élément de tige, la tige (4) étant fixée à l'extrémité avant (3) de la hampe (2),
au moins deux branches (5, 6) qui sont articulées à la tige (4), et
au moins deux mécanismes de commande (7, 9, 8, 10) flexibles, qui sont disposés au moins partiellement dans la hampe (2), dans lequel chaque branche individuelle (5, 6) peut être déplacée individuellement par un mécanisme de commande propre (7, 9, 8, 10) par rapport à la tige (4), pour former au moins deux gueules qui peuvent être ouvertes et fermées indépendamment l'une de l'autre, dans lequel
les mécanismes de commande (7, 9, 8, 10) possèdent respectivement un élément de transmission (7, 8) résistant à la traction et à la pression qui évoluent ensemble dans la hampe (2), chacun de ces éléments de transmission (7, 8) étant relié à l'extrémité avant (3) de la tige (2) à une branche (5, 6) et possédant respectivement un dispositif de commande (9, 10) qui est prévu à l'extrémité arrière (14) de la hampe (2) et est relié à l'élément de transmission associé (7, 8), dans lequel
chaque élément de transmission (7, 8) transmet le mouvement déployé par le dispositif de commande correspondant (9, 10) à la branche correspondante (5, 6),
**caractérisé en ce que** la tige (2) est flexible, **en ce que** les éléments de transmission (7, 8) sont des câbles Bowden et **en ce que** les dispositifs de commande (9, 10) sont des éléments de saisie, les éléments de saisie (9, 10) étant configurés de telle sorte qu'un actionnement des éléments de saisie (9, 10) ouvre la branche correspondante (5, 6) et qu'une libération des éléments de saisie (9, 10) ferme la branche correspondante (5, 6).

2. Appareil médical de saisie (1) selon la revendication 1,
**caractérisé en ce que**
la tige (4) consiste en au moins deux éléments de tige (4A, 4B), au maximum, l'un des éléments de tige (4A, 4B) étant fixé de façon rigide à la tige (2) et les éléments de tige restants (4A, 4B) pouvant être orientables à l'extrémité avant de la hampe (2) ou étant articulés au maximum à un élément de tige rigide (4A, 4B),
au moins un élément d'actionnement (70) étant disposé sur les éléments de tige (4A, 4B), par lequel les éléments de tige (4A, 4B) peuvent se déplacer l'un par rapport à l'autre, respectivement une branche (5, 6) étant articulée avec un élément de tige (4A, 4B) de façon à pouvoir tourner.

3. Appareil médical de saisie (1) selon la revendication 2,
**caractérisé en ce que**
l'élément d'actionnement a au moins un ressort (70) qui est disposé sur les éléments de tige (4A, 4B) de telle sorte qu'il écarte les éléments de tige (4A, 4B) sur leur extrémité distale (41A, 41B).

4. Appareil médical de saisie (1) selon la revendication 1,
**caractérisé en ce que**
les éléments de saisie (9, 10) sont prévus sur une poignée commune (15).

5. Appareil médical de saisie (1) selon la revendication 1,
**caractérisé en ce que**
les éléments de saisie (9, 10) sont prévus sur des poignées individuelles.

6. Appareil médical de saisie (1) selon l'une des revendications 1 à 5,
**caractérisé en ce que**
les branches (5, 6) et/ou les éléments de tige associés (4A, 4B) sont distinguables,
les dispositifs de commande associés (9, 10) sont distinguables, de sorte qu'une affectation claire de la branche correspondante (5, 6) au dispositif de commande associé (9, 10) soit possible.

7. Appareil médical de saisie (1) selon la revendication 6,
**caractérisé en ce que**
les branches (5, 6) et/ou les éléments de tige (4A, 4B) sont distinguables par leur couleur, leur forme et/ou leur matériau.

8. Appareil médical de saisie (1) selon l'une des revendications 1 à 7,
**caractérisé en ce que**
les branches (5, 6) et/ou les éléments de tige (4A, 4B) présentent des bords de coupe.

9. Appareil médical de saisie (1) selon l'une des revendications 1 à 8,
**caractérisé en ce que**
les branches (5, 6) et les éléments de tige (4A, 4B) sont disposés symétriquement et les branches (5, 6) peuvent tourner en direction radiale par rapport à l'axe de l'appareil de saisie (1).

10. Appareil médical de saisie (1) selon l'une des revendications 1 à 9,
**caractérisé en ce que**
les éléments de tige (4A, 4B) peuvent tourner en direction radiale par rapport à l'axe de l'appareil de saisie (1).

11. Appareil médical de saisie (1) selon l'une des revendications 1 à 10,
**caractérisé en ce que**
la tige (4) et les branches (5, 6) sont de même longueur par rapport à leur extrémité avant.
